# EUROPEAN PATENT APPLICATION

(11) **EP 0 995 433 A1**
(43) Date of publication of application: **26.04.2000**
(21) Application number: 99308011.8
(22) Date of filing: 12.10.1999
(51) Int. Cl.: A61K 7/48

(54) **Retinol stabilized cleansing compositions**

(30) Priority: 13.10.1998 US 104105 P; 14.05.1999 US 312388
(71) Applicant: JOHNSON & JOHNSON CONSUMER COMPANIES, INC., Skillman, NJ 08558 (US)
(72) Inventor: Nystrand, Glenn, Lebanon, NJ 08833 (US); Debois, Jennifer, Princeton, NJ 08540 (US)
(74) Representative: Mercer, Christopher Paul

(57) **Abstract**

The present invention provides compositions which include (A) a skin enhancing agent; (B) glycerin; (C) one or more emollients; and (D) a salt of cocoyl isethionate, a mono alkyl phosphate or a salt thereof, or a combination thereof, neutralized to a pH ranging from about 5 to about 8. In an alternate embodiment, the present invention provides compositions described above which further include (E) water; (F) a gum; (G) a gum neutralized to a pH ranging from about 5 to about 8; (H) a detergent additive selected from the group consisting of sodium lauryl sulfate, a sodium salt of fatty acid taurate, an acyl glutamate, an alpha-olefin sulfonate, a sarcosinate, or any combination of any of the foregoing; (I) a mixture of diosodium cocamido monoethanolamine sulfosuccinate and cocamido betaine; (J) an anti-microbial agent; (K) microcrystalline wax; (L) a foam booster; (M) an antioxidant; (N) a UV stabilizer; (O) a sun screen; (P) a colorant; (Q) a fragrance; (R) a preservative; (S) a chelating agent; (T) a physical exfolliant; or (U) any combination of any of the foregoing. These compositions are useful in preparing stable formulations containing pH dependent active agents including, but not limited to, retinol. Also provided are methods of delivering skin-enhancing agents, such as retinol, to an animal, preferably a mammal, and most preferably a human, by topically applying the compositions of the present invention to the skin of an animal in need of the skin enhancing agent.

## Description

### FIELD OF THE INVENTION

This invention relates to cosmetically aesthetic, stable cleansing preparations containing skin-enhancing agents such as, for example, retinol.

### BACKGROUND OF THE INVENTION

A mild depositing cleanser is disclosed in Ramirez et al., U.S. Patent No. 5,409,706. This cleanser is an essentially anhydrous oil and surfactant mixture dispersed in a continuous glycerin phase. However, this formulation exhibits poor aesthetics including excess firmness and an occasional granular texture. This formulation also exhibits poor in-use aesthetics such as, for example, poor spreadability when contacted with water (which leads to the floating chunks of the cleanser) and low amounts of foam. This cleanser also exhibits poor stability in that it often develops syneresis of both oils (at the top of the cream) and glycerin (at the bottom). Even when left at room temperature for relatively short periods of time, tube samples ooze clear liquids. See also, U.S. Patent No. 5,254,334. See also, U.S. Patent No. 5,602,087 which discloses a low water content composition in solid form.

Moreover, certain active agents such as, for example, retinol are unstable at low pH such as the pH of the given thickened compositions of U.S. Patent No. 5,409,706.

The present inventors have discovered a cleanser formulation which overcomes these deficiencies, and which provides a stable vehicle for pH dependent active agents such as retinol.

### SUMMARY OF THE INVENTION

In a first embodiment, the present invention provides compositions which include:
(A) a skin enhancing agent;
(B) glycerin;
(C) one or more emollients; and
(D) a salt of cocoyl isethionate, a mono alkyl phosphate or a salt thereof, or a combination thereof, neutralized to a pH ranging from about 5 to about 8.

In an alternate embodiment, the present invention provides compositions described above which further include:
(E) water;
(F) a gum;
(G) a gum neutralized to a pH ranging from about 5 to about 8;
(H) a detergent additive selected from the group consisting of sodium lauryl sulfate, a sodium salt of fatty acid taurate, an acyl glutamate, an alpha-olefin sulfonate, a sarcosinate, or any combination of any of the foregoing;
(I) a mixture of diosodium cocamido monoethanolamine sulfosuccinate and cocamido betaine;
(J) an anti-microbial agent;
(K) microcrystalline wax;
(L) a foam booster;
(M) an antioxidant;
(N) a UV stabilizer;
(O) a sun screen;
(P) a colorant;
(Q) a fragrance;
(R) a preservative,
(S) a chelating agent;
(T) a physical exfolliant; or
(U) any combination of any of the foregoing.

These compositions are useful in preparing stable formulations containing pH dependent active agents including, but not limited to, retinol.

Also provided are methods of delivering skin-enhancing agents, such as retinol, to an animal, preferably a mammal, and most preferably a human, by topically applying the compositions of the present invention to the skin of an animal in need of the skin enhancing agent.

### DETAILED DESCRIPTION OF THE INVENTION

The compositions of the present invention are typically creams, gels, or liquid.

Emollients suitable for use in the compositions of the present invention include, but are not limited to, petrolatum; mineral oils; and esters such as, for example, isopropyl myristate, isopropyl palmitate, 1-Decene polymer (hydrogenated), C₁₂-C₁₅ alcohol benzoates. and C₁₂-C15 alkyl benzoates esters.

Salts of cocoyl isethionate useful herein include, but are not limited to, sodium cocoyl isotheionate and ammonium cocoyl isethionate. This salt should also be neutralized to a pH ranging from about 5 to about 8, preferably from about 6.5 to about 7.5, and most preferably from about 6.8 to about 7.2. Neutralizing agents include both organic and inorganic bases. Special mention is made of triethanolamine. Neutralization of any components described as neutralized in this composition is preferably at these pH ranges, using the neutralizing agents described above.

Suitable monoalkyl phosphates include, but are not limited to, C₉-C₁₅ alkyl phosphates such as, for example, Arlatone MAP concentrate (ICI Surfactants-Wilmington, DE), lauryl phosphate acid, and Rhodafac DV4922 (Rhodia-NJ). The monoalkyl phosphate should be neutralized as described above.

Suitable gums for use herein include, but are not limited to, xanthan gum, an acrylate/C₁₀-C₃₀ cross polymer such as that sold under the trade name Carbomer 1382 by B. F. Goodrich - Cleveland OH and carboxypolymethylene polymers (Carbopol). Although xantham gum does not need to be neutralized, gums such as Carbomer should be neutralized as described above. Preferably, the concentration of base is such that the gum can be neutralized by about a 1:1 weight ratio of gum.

Detergent additives useful herein are selected from the group of detergents consisting of sodium lauryl sulfate, sodium salts of fatty acid taurate, acyl glutamates, α-olefin sulfonates, and sarcosinates such as, for example, sodium lauryl sarcosinate.

Additionally, a combination of disodium cocamido MEA surfactant and cocamidopropyl betaine such as that sold as Mackadet CMB100 (McIntyre Chemicals-University Park- IL) can be added to the present compositions. Retinol delivery enhancers such as, for example, C₁₂-C₁₅ alkyl benzoates may also be added. A small amount of microcrystalline was may be added to produce a cosmetically acceptable cream.

Additionally, foam boosters may be incorporated into the compositions of the present invention. Suitable foam enhancers include, but are not limited to, potassium polymetaphosphate, n-pentane, isopentane, sodium lauryl amides. These materials will enhance the foam produced when the present compositions are exposed to water during use.

The compositions of this invention may also contain additives such as fragrances, colorants, sugar, and sugar derivatives to improve the texture, appearance and user perception.

Skin-enhancing agents suitable for use in the present invention are those having a positive effect on the skin such as a sunscreen, an exfolliatory agent, or retinol. Retinol is vitamin A alcohol. Retinol stability is pH dependent and requires a pH ranging from about 5 to about 8. Special mention is also made of tretinoin which is vitamin A acid of the cream. Tretinoin does not need to be neutralized and could have a pH range from about 4 to about 8 and preferably from about 4 to about 5. Additional active ingredients may be incorporated in the present compositions. Such active ingredients include, but are not limited to deodorants, medicaments such as, for example, coal-tar, benzoyl peroxide, vitamin A and vitamin E and antibacterial or anti-microbial ingredients such as, for example, triclosan, PV-iodine and salicylic acid. Other additives such as, for example, antioxidants, UV stabilizers, and sun screens may also be included.

The amounts of each of the components of the present compositions are typically those amounts effective to accomplish the purpose of that component.

Water, if added, is typically added in an amount of about 5 percent by weight or greater based upon 100 percent by weight of total composition. Preferably, the amount of water ranges from about 5 to about 20 percent by weight, and most preferably from about 5 to about 15 percent by weight or from about 5 to about 12 percent by weight on the same basis.

The compositions of the present invention typically contain skin enhancing agent(s) such as, for example retinol, in an amount ranging from about 0.1 to about 2 percent by weight; preferably from about 0.5 to about 1 percent by weight, and most preferably about 0.5 percent by weight (special mention is made of retinol in an amount of from about 0.1 to about 0.2 percent by weight); glycerin in an amount ranging from about 30 to about 60 percent by weight, preferably from about 35 to about 50 percent by weight, and most preferably, from about 45 to about 46 percent by weight; neutralized sodium cocoyl isethionate in an amount ranging from about 3 to about 19 percent by weight, preferably from about 5 to about 15 percent by weight, and most preferably about 7.2 percent by weight; neutralized monoalkylphosphate or salt thereof in an amount ranging from about 0.5 to about 5 percent by weight, preferably from about 1 to about 3 percent by weight, and most preferably about 1 percent by weight; emollients in an amount ranging from about 20 to about 45 percent by weight, preferably from about 25 to about 35 percent by weight, and most preferably about 32 percent by weight, based upon 100 percent by weight of the composition.

The amount of detergent additive may be an effective amount for increasing the softness of the composition.

Preferably, the weight ratio of detergent additive to cocoyl isethionate salt ranges from about 1:6 to about 1:1. While the precise amount of detergent employed will depend upon the particular detergent employed, normally the detergent additive will be present in an amount ranging from about one to about 20 weight percent, based upon 100 percent by weight of total composition. For example, when sodium lauryl sulfate is used as the detergent additive, an effective amount will range from about 1 to about 5 percent by weight. Preferably, the weight ratio of sodium lauryl sulfate to cocoyl isethionate salt in the composition ranges from about 1:3 to about 1:6.

Effective amounts, based upon 100 percent by weight of total composition, of other detergent additives are as follows:

| **Detergent Additive** | **Effective Amount** |
|---|---|
| Sodium salts of fatty acid taurate | about 1 to about 5 weight percent |
| Acyl glutamates | about 1 to about 19 weight percent |
| α-olefin sulfonates | about 1 to about 15 weight percent |

Typically, microcrystalline wax will be present in an amount ranging from about 4 to about 10 percent by weight, based upon 100 percent by weight of total composition.

The compositions of the present invention may be prepared by vigorously mixing the ingredients together. The order or addition is not critical. An example of one way of making the claimed composition is as follows.

### Batch Process:

### Glycerin Phase

1. Add the glycerin to suitable size beaker and agitate.
2. Sift in Carbomer 1382, methylparaben, propylparaben, and Versene XL100 (tetrasodium EDTA) and continue to mix.
3. Transfer to homogenizer and begin to homogenize and heat to 60°C.
4. Agitate until polymer slurry is uniform (no fish eyes evident).
5. Add Deionized Water and adjust temperature to 80°C.
6. Neutralize with Triethyanolamine 99%. Maintain at 80°C and continue to agitate on homogenizer. Hold for transfer to Esco vessel.

### Oil Phase Part A

1. Add Petrolatum to a suitable sized beaker and begin to heat to 80°C.
2. When Petrolatum begins to soften and liquefy begin to homogenize (low setting) with a gifford wood homogenizer.
3. Add Arlatone MAP Acid and neutralize with the Trolamine 99% (triethanolamine). Homogenize.
4. Add Tauranol I 78 and allow to wet out in melted oils. Maintain agitation (homogenizer) and heat at 80°C.
5. Add Bio-Terge (sodium α-olefin-sulfonate) (NaC₁₄-C₁₆ olefin-sulfonate) AS 90 and allow to wet out in melted oils. Maintain agitation (homogenizer) and heat at 8o°C.
6. Add Potassium Polyphosphate. Maintain homogenizer agitation and temperature at 80°C.
7. Add Titanium Dioxide and mix until uniform. Maintain homogenizer agitation and temperature at 80°C.

### Oil Phase Part B

1. In a separate breaker, heat Finsolv (C₁₂-C₁₅ alkyl benzoate) TN to 80°C.
2. Add BHT to the Finsolv, mix and allow to dissolve, then add Retinol Soybean mixture and allow to completely dissolve.
3. Hold at 75-80°C for phasing to the Esco Vessel.

### Vacuum Vessel

1. Transfer the Oil Phase Part A components (80°C) to the vessel which is present at 80°C. Start mixing with side scrapper agitation set at 20-40% of max. speed. (Actual =35%)
2. Draw a vacuum of -0.6 - 0.8 bar. (Actual = -0.7 bar).
3. Transfer the Glycerin phase components (80°C) to funnel hopper and add to the glycerin phase while continuing to mix. Mix with sweep for 5 minutes. Maintain a vacuum of -0.6 to -0.8 bar.
4. Transfer the Oil Phase Part B components (80°C) to the vessel which is preset at 80°C. Start the homogenizer, and homogenize for 10 minutes at 40% of maximum.
5. If necessary adjust vacuum to -0.6 to 0.8 bar and maintain heat at 80°C for 10-15 minutes.
6. Check the batch visually for a uniform appearance. Maintain vacuum, low to moderate (25-35) actual = 30, sweep speed, and low (20-25) actual =20, homogenizer, and cool the batch to 45°C.
7. When the batch is at 45°C turn off the homogenizer and maintain sweep agitation. Add the fragrance and allow to mix for 20 minutes at 40-45°C.
8. Cool to 35°C with sweep agitation and fill.

These compositions are administered topically to an animal in need of any active or skin enhancing agent contained therein. Typically, the amount administered is a skin-enhancing effective amount.

### DESCRIPTION OF THE PREFERRED EMBODIMENTS

The following examples illustrate the invention without limitation.

### Example 1

The composition was prepared having a formulation as in Table 1 below. This composition included a physical exfolliating agent.

**Table 1**

| **CTFA Name** | **%Wt** |
|---|---|
| **GLYCERIN PHASE** | |
| Glycerin | 44.8 |
| Deionized Water | 10 |
| Acrylates/C10-30 Alkyl Crosspolymer | 0.15 |
| Triethanolamine | 0.15 |

| **OIL PHASE** | |
|---|---|
| Petrolatum | 25 |
| C12-15 Alkyl Benzoate | 7 |
| C9-15 Alkyl Phosphate Acid | 0.63 |
| Triethanolamine | 0.38 |
| BHT | 0.1 |
| Sodium Cocoyl Isethionate | 6.8 |
| Sodium C14-16 Olefin Sulfonate | 1.2 |
| Potassium Polyphosphate | 0.5 |
| Titanium Dioxide | 0.3 |

| **BEAD PHASE** | |
|---|---|
| Hydrogenated Jojoba Oil | 3 |
| | **100** |

### Example 2

A composition was prepared having a formulation as in Table 2 below.

**Table 2**

| **Trade Ingredient** | **CTFA Name** | **%Wt** |
|---|---|---|
| **GLYCERIN PHASE** | | |
| Glycerin 916 USP | Glycerin | 56.45 |
| Carbopol 1382 | Acrylates/C10-30 Alkyl Crosspolymer | 0.15 |
| Trolamine 89% | Triethanolamine | 0.75 |

| **OIL PHASE Part A** | | |
|---|---|---|
| Petrolatum G1951 | Petrolatum | 25 |
| Aralone MAP Conc. | C9-C15 Alkyl Phosphate | 6.25 |
| Trolamine 98% | Triethanolamine | 3.75 |
| Potassium Polymetaphosphate Powder | Potassium Polyphosphate | 0.5 |
| Titanium Dioxide | Titanium Dioxide | 0.3 |
| Finsolv TN | C12-15 Alkyl Benzoate | 6.75 |
| BHT | BHT | 0.1 |
| | | **100** |

### Example 3

A composition was prepared having a formulation as in Table 3 below.

**Table 3**

| **Trade Ingredient** | **CTFA Name** | **%Wt** |
|---|---|---|
| Glycerin 916 USP | Glycerin | 58.36 |
| Petrolatum G1951 | Petrolatum | 26.52 |
| Triethanolamine | Triethanolamine | 1.06 |
| Tauranol 78 | Sodium Cocoyl Isethionate | 8.49 |
| Retinol 10S (about 10% Retinol Dispersion in Soybean Oil) J&J Assay - 9.5% | Vitamin A Alcohol (and Soybean Oil | 5.57 |
| | | **100** |

### Example 4

A composition was prepared having a formulation as in Table 4 below.

**Table 4**

| **CTFA Name** | **%Wt** |
|---|---|
| **GLYCERIN PHASE** | |
| Glycerin | 57.25 |
| Acrylates/C10-30 Alkyl Crosspolymer | 0.15 |
| Triethanolamine | 0.75 |

| **OIL PHASE Part A** | |
|---|---|
| Petrolatum | 25 |
| C9-C15 Alkyl Phosphate Acid | 0.625 |
| Triethanolamine | 0.375 |
| Sodium Cocoyl Isethionate | 7.2 |
| Potassium Polyphosphate | 0.5 |
| Titanium Dioxide | 0.3 |

| **OIL PHASE Part B** | |
|---|---|
| C12-C15 Alkyl Benzoate | 2.5 |
| BHT | 0.1 |
| Vitamin A Alcohol (and) Soybean Oil | 5.25 |
| | **100** |

### Example 5

A composition was prepared having a formulation as in Table 5 below.

**Table 5**

| **CTFA Name** | **%Wt** |
|---|---|
| **GLYCERIN PHASE** | |
| Glycerin | 47.25 |
| Acrylates/C 10-30 Alkyl Crosspolymer | 0.15 |
| Triethanolamine | 0.75 |
| Water | 10 |

| **OIL PHASE Part A** | |
|---|---|
| Petrolatum | 25 |
| C9-C15 Alkyl Phosphate Acid | 0.625 |
| Triethanolamine | 0.375 |
| Sodium Cocoyl Isethionate | 7.2 |
| Potassium Polyphosphate | 0.5 |
| Titanium Dioxide | 0.3 |

| **OIL PHASE Part B** | |
|---|---|
| C12-C15 Alkyl Benzoate | 2.5 |
| BHT | 0.1 |
| Vitamin A Alcohol (and) Soybean Oil | 5.25 |
| | **100** |

### Example 6

A composition was prepared having a formulation as in Table 6 below. This composition included a sunscreen.

**Table 6**

| **CTFA Name** | **%Wt** |
|---|---|
| **GLYCERIN PHASE** | |
| Glycerin | 46.05 |
| Acrylates/C 10-30 Alkyl Crosspolymer | 0.15 |
| Triethanolamine | 0.75 |
| Water | 10 |

| **OIL PHASE** | |
|---|---|
| Petrolatum | 12.75 |
| C9-C15 Alkyl Phosphate Acid | 0.625 |
| Triethanolamine | 0.375 |
| Sodium Cocoyl Isethionate | 7.2 |
| Potassium Polyphosphate | 0.5 |
| Titanium Dioxide | 0.3 |
| Sodium C14-16 Olefin Sulfonate | 1.2 |
| BHT | 0.1 |
| Octyl Methoxycinnamate | 20 |
| | **100** |

### Example 7

A composition was prepared having a formulation as in Table 7 below.

**Table 7**

| **CTFA Name** | **%Wt** |
|---|---|
| **GLYCERIN PHASE** | |
| Glycerin | 57.05 |
| Acrylates/C 10-30 Alkyl Crosspolymer | 0.15 |
| Triethanolamine | 0.75 |

| **OIL PHASE Part A** | |
|---|---|
| Petrolatum | 25 |
| Disodium Cocamido MEA Sulfosuccinate (and) Cocamidopropyly Betaine | 1.2 |
| Sodium Cocoyl Isethionate | 7.2 |
| Potassium Polyphosphate | 0.5 |
| Titanium Dioxide | 0.3 |

| **OIL PHASE Part B** | |
|---|---|
| C12-C15 Alkyl Benzoate | 2.5 |
| BHT | 0.1 |
| Vitamin A Alcohol (and) Soybean Oil | 5.25 |
| | **100** |

### Example 8

A composition was prepared having a formulation as in Table 8 below:

**Table 8**

| **CTFA Name** | **%Wt** |
|---|---|
| **GLYCERIN PHASE** | |
| Glycerin | 45.35 |
| Acrylates/C10-30 Alkyl Crosspolymer | 0.15 |
| Triethanolamine | 0.75 |
| Methylparaben | 0.2 |
| Propylparaben | 0.1 |
| Tetrasodium EDTA | 0.2 |
| Water | 10 |

| **OIL PHASE Part A** | |
|---|---|
| Petrolatum | 25 |
| C9-C15 Alkyl Phosphate Acid | 0.625 |
| Triethanolamine | 0.375 |
| Sodium Cocoyl Isethionate | 7.2 |
| Sodium C14-16 Olefin Sulfonate | 1.2 |
| Potassium Polyphosphate | 0.5 |
| Titanium Dioxide | 0.3 |

| **OIL PHASE Part B** | |
|---|---|
| C12-C15 Alkyl Benzoate | 2.5 |
| BHT | 0.1 |
| Vitamin A Alcohol (and) Soybean Oil | 5.25 |
| **Fragrance** | |
| Fragrance | 0.2 |
| | **100** |

All patents, publications, applications, and test methods mentioned herein are hereby incorporated by reference.

Many variations of the present invention will suggest themselves to those skilled in the art in light of the above, detailed description. All such obvious variations are within the full intended scope of the appended claims.

## Claims

1. A composition comprising:
(A) a skin enhancing agent;
(B) glycerin,
(C) one or more emollients; and
(D) a salt of cocoyl isethionate, a mono alkyl phosphate or a salt thereof, or a combination thereof, neutralized to a pH ranging from 5 to 8.

2. The composition of claim 1, wherein said skin-enhancing agent is retinol.

3. The composition of claim 1, wherein said skin-enhancing agent is tretinoin.

4. The composition of any one of claims 1 to 3, further comprising any one of: (E) water; (F) a gum; (G) a gum neutralized to a pH ranging from about 5 to about 8; (H) a detergent additive selected from the group consisting of sodium lauryl sulfate, a sodium salt of fatty acid taurate, an acyl glutamate, an alpha-olefin sulfonate, a sarcosinate, or any combination of any of the foregoing; (I) a mixture of diosodium cocamido monoethanolamine sulfosuccinate and cocamido betaine; (J) an anti-microbial agent; (K) microcrystalline wax; (L) a foam booster; (M) an antioxidant; (N) a UV stabilizer; (O) a sun screen; (P) a colorant; (Q) a fragrance; (R) a preservative; (S) a chelating agent; (T) a physical exfoliating agent; or (U) any combination of any of the foregoing.

5. The composition of any one of claims 1 to 4, wherein said one or more emollients is petrolatum, a mineral oil, a 1-decene polymer (hydrogenated), an ester or a mixture thereof.

6. The composition of claim 5, wherein said one or more emollients is isopropyl palmitate, isopropyl myristate, C₁₂-C₁₅ alcohol benzoate or a mixture thereof.

7. The composition of any one of claims 1 to 6, wherein said monoalkyl phosphate or salt thereof is a mono C₉-C₁₅ alkyl phosphate or a salt thereof.

8. The composition of any one of claims 1 to 7, wherein said salt of cocoyl isethionate is sodium cocoyl isethionate.

9. The composition of any one of claims 1 to 8, wherein said salt of cocoyl isethionate, monoalkyl phosphate or salt thereof, or combination thereof, is neutralized to a pH ranging from 6.5 to 7.5, preferably from 6.8 to 7.2.

10. The composition of claim 2, or any claim dependent thereon, wherein the amount of retinol ranges from 0.1 to 0.2 percent by weight, based upon 100 percent by weight of total composition.
